(19) Europäisches Patentamt
European Patent Office
Office européen des brevets

(11) Veröffentlichungsnummer: **0 028 609**
**B1**

(12) # EUROPÄISCHE PATENTSCHRIFT

(45) Veröffentlichungstag der Patentschrift: **27.07.83**

(21) Anmeldenummer: **80900764.4**

(22) Anmeldetag: **08.05.80**

(86) Internationale Anmeldenummer: **PCT/CH 80/00056**

(87) Internationale Veröffentlichungsnummer: **WO 80/02493 (27.11.80** Gazette **80/27)**

(51) Int. Cl.³: **A 01 N 33/18,** A 01 N 37/48, A 01 N 43/50, C 07 C 79/22, C 07 C 79/36, C 07 C 79/46, C 07 C 103/22, C 07 C 121/68, C 07 C 109/10, C 07 D 233/44

(54) **Verwendung von Chlornitrophenylderivaten als selektive Herbizide und neue Chlornitrophenylderivate.**

(30) Priorität: **16.05.79 CH 4548/79**

(43) Veröffentlichungstag der Anmeldung: **20.05.81 Patentblatt 81/20**

(45) Bekanntmachung des Hinweises auf die Patenterteilung: **27.07.83 Patentblatt 83/30**

(84) Benannte Vertragsstaaten: **DE FR**

(56) Entgegenhaltungen:
**BE-A-760 968**
**FR-A-1 291 128**
**FR-A-1 568 656**
**GB-A-1 246 305**
**US-A-3 009 806**
**US-A-3 474 090**
**US-A-3 547 619**
**Chemical Abstracts, Band 77, Nr. 3, 17. Juli 1972, Columbus, Ohio, US, Chamberlain V. H. u. a.: »Plant growth regulating substances, XXXIII Influence of ring substituents on the plant growth regulating activity of phenylacetic acid«, Zusammenfassung Nr. 15433g Seite 166, siehe das ganze Artikel**

(73) Patentinhaber: **SIEGFRIED AKTIENGESELLSCHAFT, CH-4800 Zofingen (CH)**

(72) Erfinder: **THIELE, Kurt, Rebbergstrasse 47d., CH-4800 Zofingen (CH)**
Erfinder: **GEISSMANN, Felix, Brittnauerstrasse 28, CH-4800 Zofingen (CH)**
Erfinder: **MOLNAR, Istvan, Eschenweg 10, CH-4800 Zofingen (CH)**
Erfinder: **SCHÄREN, Walter, Lärchenfeld 440, CH-4803 Vordemwald (CH)**
Erfinder: **MEYER, Jacques, Obere Brühlstrasse 1, CH-4800 Zofingen (CH)**

(74) Vertreter: **Ritscher, Thomas, Dr. et al, RITSCHER & SEIFERT Auf der Mauer 4, CH-8001 Zürich (CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



BUNDESDRUCKEREI BERLIN

# Verwendung von Chlornitrophenylderivaten als selektive Herbizide und neue Chlornitrophenylderivate

Chlornitrophenylderivate mit herbizider Aktivität sind in großer Zahl seit längerem bekannt, z. B. aus den DE-AS 1 115 514 und 1 285 248 sowie der Arbeit von H. E. Thompson et al in Botan. Gaz. 107, 1946, 476—507 (siehe C. A. 41, 1947, 3902—3912, in der über tausend spezielle Verbindungen angegeben sind).

In Arbeiten von M. P. Pybus et al (Ann. appl. Biol. 47, 1959, 593—600) und V. H. Chamberlain et al (Ann. appl. Biol. 69, 1971, 65—72 und dort genannten Literaturstellen) sind ferner die unterschiedlichen wachstumsregulierenden Aktivitäten der Phenylessigsäure und von nitrohalogen-, methyl-, amino-, hydroxyl- und acetylamino-substituierten Derivaten dieser Säure mit der Formel (10)

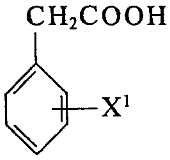

(10)

untersucht worden, in der $X^1$ ein bis drei gleiche oder unterschiedliche Substituenten, und zwar Nitro-, Halogen-, Methyl-, Amino-, Hydroxyl- oder N-Acetylaminogruppen bedeutet.

In der erstgenannten Arbeit wurde aufgrund der hohen Aktivitäten der 2,3-Dichlor- und der 2,6-Dichlorphenylessigsäure die 2,3,6-Trichlorphenylessigsäure untersucht und als außerordentlich aktiv befunden; gemäß der zweitgenannten Arbeit erweist sich die 2,4-Dinitro- sowie die 2,6-Dinitrophenylessigsäure als vergleichsweise wenig aktiv, und ist die 3-Nitrophenylessigsäure deutlich wirksamer als die 2-Nitrophenylessigsäure. Schließlich wurden auch die verschiedenen Chlornitrophenylessigsäuren untersucht, nämlich die 3-Nitroverbindungen mit Chlor in 2-, 4-, 5- und 6-Stellung, sowie die 2-Nitroverbindungen mit Chlor in 4-, 5- und 6-Stellung. Daraus wurde für die Nitrochlorphenylessigsäuren folgende Aktivitätsskala abgeleitet:

$$2-Cl-3-NO_2- > 2-Cl-6-NO_2- > 4-Cl-3-NO_2- > 6-Cl-3-NO_2.$$

Erstaunlicherweise ist die an sich, aber nicht als Herbizid, sondern nur als eigenschaftsloses Zwsichenprodukt von Pyrrolsynthesen (S. Umio et al, Chem. Pharm. Bull. 17, 1969, 605—610) bekannte 2-Nitro-3-chlorphenylessigsäure in den obengenannten systematischen Untersuchungen nicht behandelt und der gesamte Stand der Technik enthält keinerlei Hinweis auf herbizide Eigenschaften von 2-Nitro-3-chlor-phenylderivaten.

Aus US-A-3 547 619 sind herbizide bzw. selektiv herbizide Phenylmethylencarbonylderivate mit 2ständiger Nitrogruppe bekannt, die gegebenenfalls einen weiteren ringständigen Substituenten in 6-Stellung tragen können, wobei der 6-Substituent gewählt ist aus Methyl, Wasserstoff und Chlor.

Abgesehen davon, daß die 3-Stellung dieser bekannten Phenylverbindungen stets unsubstituiert ist, läßt sich aus diesen Verbindungen weder die Notwendigkeit einer zweifachen Substitution noch die kritische Bedeutung von Chlor als 3-Substituent ableiten. Ferner ist hier mindestens die Carbonylgruppe der Phenylessigsäurestruktur kritisch.

Neu ist daher der Befund, daß die 2-Nitro-3-chlorphenylessigsäure und andere 2-Nitro-3-chlorphenylderivate eine herbizide Wirkung haben, und es kann als überraschend gelten, daß diese Wirkung nicht nur unabhängig von der Carboxyl- bzw. Carbonylgruppe ist, sondern auch eine bemerkenswerte Selektivität, insbesondere bei Vorlauf/Nachlauf-Anwendung auf monokotyle bzw. dikotyle Planzen hat. Weitere Untersuchungen der Anmelderin führten dann zu der ausweislich des genannten Standes der Technik überraschenden Erkenntnis, daß bei 2-Nitro-3-chlorphenylderivaten die Essigsäuregruppe für die herbizide Aktivität nicht kritisch ist und beispielsweise durch die Acetamido-, die Acetonitril- und sogar durch die Ethanolgruppe ersetzt werden kann, und daß dabei im Vergleich zur Säure nicht nur gleichwertige, sondern sogar verbesserte Herbizidaktivitäten erzielbar sind.

Gegenstand der Erfindung ist die Verwendung von Verbindungen der Formel (1)

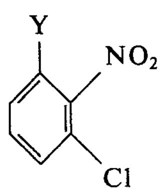

(1)

als selektive Herbizide, wobei in Formel (1) Y eine Gruppe der Formeln (1a), (1b) oder (1c)

$-CH_2CH_2-OR^1$ 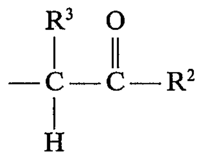 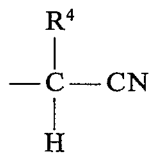

(1a) (1b) (1c)

darstellt, in welchen $R^1$ das Wasserstoffatom, oder der Säurerest einer $C_{1-6}$-Alkansäure ist, die gegebenenfalls einen $C_{6-12}$-Aryl-, $C_{6-12}$-Aryloxy-, $C_{6-12}$-Ar-$C_{1-18}$-alk-$C_{6-12}$-aryl- oder $C_{6-12}$-Ar-$C_{1-18}$-alk-$C_{6-12}$-aryloxyrest trägt, dessen Arylteil durch Halogen und/oder Nitro substituiert sein kann, $R^2$ das Wasserstoffatom; die Gruppe $-OZ$, in welcher Z das Wasserstoffatom, den einwertigen Rest eines Kations oder einen gegebenenfalls durch Halogen und/oder Nitro substituierten $C_{1-18}$-Alkyl-, $C_{6-12}$-Aryl-, $C_{7-18}$-Alkaryl- oder $C_{7-18}$-Aralkylrest bedeutet; eine Gruppe der Formel

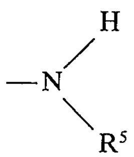

in welcher $R^5$ das Wasserstoffatom, die Aminogruppe, einen gegebenenfalls durch Halogen oder Nitro substituierten $C_{1-18}$-Alkylrest oder einen alicyclischen oder heteroaromatischen Rest mit 5—6 Ringatomen darstellt; oder ein gegebenenfalls durch Halogen und/oder Nitro substituierter $C_{1-18}$-Alkyl-, $C_{6-12}$-Aryl-, $C_{7-18}$-Alkaryl- oder $C_{7-18}$-Aralkylrest ist; und $R^3$ und $R^4$ Wasserstoffatome, Niederalkylgruppen ($C_{1-4}$) oder Halogenatome sind.

Als $R^1$ werden insbesondere solche Säurereste der genannten Gruppen bevorzugt, die in Form der Säuren Herbizidwirkung haben, wie z. B. die Benzylphenoxyalkancarbonsäuren der in der DE-A-2 417 487 beschriebenen Art; ein spezielles Beispiel ist der Säurerest der in der DE-A-2 461 069 von der Anmelderin als Lipidsenker beschriebenen p-(p'-Chlorbenzyl)-phenoxyessigsäure. Ebenfalls als Säuren für Säurereste als $R^1$ sind die im eingangs genannten Stand der Technik beschriebenen herbiziden Säuren, einschließlich der substituierten Benzoesäuren, der (2,4-Dichlorphenoxy)-essigsäure, der substituierten Phenylessigsäuren mit bekannter Pflanzenwachstumsaktivität und der bisher nicht als Herbizid vorbeschriebenen 2-Nitro-3-chlorphenylessigsäure.

Als Beispiele heterocyclischer Reste mit 5—6 Ringatomen für $R^5$ sind der Imidazol-, Pyrazol-, Thiazol-, Pyridin-, Pyrazin-, Pyrazolin-, Pyrimidin- oder Pyridazinrest zu nennen.

Beispiele für Verbindungen der Formel (1) mit der Gruppe (1b) als Y sind außer der 2-Nitro-3-chlorphenylessigsäure somit die Salze dieser Säure mit anorganischen oder organischen Basen, wie NaOH, KOH, $Ca(OH)_2$, $Mg(OH)_2$, $Al(OH)_3$, $NH_4OH$ und Aminiumbasen, die Ester dieser Säure mit entsprechenden Alkanol- bzw. Phenolderivaten, die gegebenenfalls N-substituierten Amide der Säure und die gemäß obigen Angaben oder in analoger Weise substituierten Derivate hiervon.

Gegenstand der Erfindung sind ferner die neuen Verbindungen der oben angegebenen Formel (1), bei welchen Y eine Gruppe der Formeln (1a), (1b) oder (1c)

$-CH_2CH_2-OR^1$ 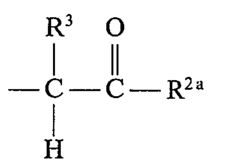 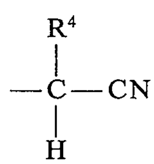

(1a) (1b) (1c)

darstellt, in welchen $R^1$, $R^3$ und $R^4$ die oben angegebene Bedeutung haben, und $R^{2a}$ die Aminogruppe oder eine N-Monoalkylaminogruppe ist, deren Alkylteil 1—6 C-Atome enthält und gegebenenfalls durch Amino, Halogen oder Hydroxyl substituiert ist.

Die erfindungsgemäßen Verbindungen sind als Herbizide und/oder als Zwischenverbindungen für die Synthese von Herbiziden oder anderen biologisch aktiven Verbindungen einschließlich von Agrochemikalien und Arzneiwirkstoffen geeignet.

Die an sich bekannte 2-Nitro-3-chlorphenylessigsäure kann nach der in der obengenannten Arbeit von S. Umio et al beschriebenen Methode hergestellt werden. Die von dieser Säure ableitbaren neuen Salze, Ester, Amide sowie der entsprechende Ethanol und das Acetonitril können aus der Säure nach an sich bekannten Verfahren erhalten werden.

Die Herstellung von bevorzugten Verbindungen wird in den folgenden Beispielen beschrieben, in denen alle Angaben in Prozent bzw. Teilen auf das Gewicht bezogen sind.

Beispiel 1

2-Nitro-3-chlorphenylessigsäure

(Formel 1, Y = $-CH_2COOH$)

70 g 2-Nitro-3-chlorbrenztraubensäure (Formel 1, Y = $-CH_2C(O)COOH$) wurden entsprechend der Arbeitsweise von Chem. Pharm. Bull. 17, 1969, 609, in 580 ml einer 0,5 n wäßrigen Natriumhydroxidlösung gelöst und bei 10°C (Kühlung) tropfenweise mit 280 ml einer 15%igen wäßrigen $H_2O_2$-Lösung versetzt. Dabei entfärbte sich die tiefrote Lösung. Nach 2 Std. Stehen bei Raumtemperatur wurde die Reaktionslösung mit 10%iger wäßriger Salzsäure angesäuert. Der gebildete Niederschlag wurde abgenutscht, mit Wasser gewaschen und getrocknet. Es wurden 60 g 2-Nitro-3-chlorphenylessigsäure, F = 161 — 162° C, entsprechend einer Ausbeutet von 90% erhalten.

Analyse
berechnet für $C_8H_6ClNO_4$: C 44,57, H 2,80, N 6,50, Cl 16,45
gefunden: C 44,13, H 2,80, N 6,30, Cl 16,97

Die als Ausgangsmaterial verwendete 2-Nitro-3-chlorbrenztraubensäure kann wie folgt erhalten werden:

157 g 2-Nitro-3-chlortoluol (hergestellt durch Oxidation von technisch erhältlichem 2-Chlor-6-methylanilin nach der Methode gemäß Houben-Weyl, 10/1, Seite 837) werden zusammen mit 137 g frisch destilliertem Oxalsäurediethylester zu einer mit 1350 ml absolutem Diethyläther verdünnten Lösung aus 80 g Kaliumethylat in 220 ml absolutem Ethanol (hergestellt durch Eintragen des Kaliumethylates unter Stickstoff in das Ethanol) gegeben. Die entstehende dunkelrote Lösung wird 28 Std. unter Rückfluß gekocht. Nach dem Abkühlen wird das ausgeschiedene Enolsalz abgenutscht und mit absolutem Diethyläther gewaschen. Die Mutterlauge wird auf 1/10 Vol eingeengt und zur Gewinnung von weiterem Enolsalz filtriert. Die Gesamtausbeute an Enolsalz beträgt etwa 70 Gew.-%. Das Enolsalz wird nach dem Trocknen 1 Std. in 1 Liter 0,5 n wäßriger KOH-Lösung gerührt. Die entstandene dunkelbraune Lösung wird zweimal mit Diethyläther extrahiert, dann mit konzentrierter Salzsäure angesäuert und mit Diethyläther extrahiert. Der so gewonnene Ätherextrakt wird getrocknet und dann eingedampft. Durch Umristallisieren des erhaltenen Rückstands aus Benzol/Äther (1 : 1 Volumteile) werden 100 g 2-Nitro-3-chlorphenylbrenztraubensäure, F = 159—161° C, entsprechend einer Ausbeute von 65% der Theorie erhalten.

Beispiel 2

Natriumsalz der 2-Nitro-3-chlorphenylessigsäure

(Formel 1, Y = $-CH_2COO^-Na^+$)

Die nach Beispiel 1 erhaltene 2-Nitro-3-chlorphenylessigsäure wurde in äquimolaren Anteilen mit wäßriger Natriumbicarbonatlösung umgesetzt. Das so entstandene Salz wurde in einer Ausbeute von 80% durch Umkristallisieren gewonnen, F = 223° C.

Beispiel 3

Dimethylaminsalz der 2-Nitro-3-chlorphenylessigsäure

(Formel 1, Y = $-CH_2COO^-(H_2)N^+(CH_3)_2$

8 g der nach Beispiel 1 erhaltenen 2-Nitro-3-chlorphenylessigsäure wurden in 500 ml wasserfreiem Diethyläther gelöst. In die Lösung wurde während 8 min trockenes gasförmiges Dimethylamin eingeleitet. Die ausgefallenen Kristalle wurden abgenutscht und mit Diethyläther gewaschen. Es wurden 8,5 des gewünschten Salzes, F = 78—79° C, erhalten.

Beispiel 4

2-Nitro-3-chlorphenylessigsäure-methylester

(Formel 1, Y = $-CH_2COOCH_3$)

Eine Mischung aus 50 g der 2-Nitro-3-chlorphenylessigsäure (gemäß Beispiel 1), 100 ml Methanol, 1000 ml Benzol und eine geringe Menge konzentrierter Schwefelsäure wurde 22 Std. Unter Rückfluß gekocht, dann abgekühlt und in der Folge mit Wasser sowie mit 2 n wäßriger Sodalösung extrahiert. Die organische Phase wurde getrocknet und nach dem Einengen aus Cyclohexan umkristallisiert. Es wurden 40 g des Zielesters, F = 61—62° C, erhalten.

Beispiel 5

2-Nitro-3-chlorphenylessigsäure-n-butylester

(Formel 1, Y = $-CH_2COOCH_2CH_2CH_2CH_3$)

Dieser Ester wurde ähnlich wie in Beispiel 4 aus der Säure von Beispiel 1 und überschüssigem n-Butanol unter Verwendung einer katalytisch aktiven Menge p-Toluolsulfonsäure verestert. Das Produkt wurde unter vermindertem Druck destilliert. Der n-Butylester wurde als hellgelbes Öl, $Kp_{0,02}$ = 130° C, in einer Ausbeute von etwa 60% erhalten.

Beispiel 6

2-(2-Nitro-3-chlorphenyl)-ethanol

(Formel 1, Y = $-CH_2CH_2OH$)

2-Nitro-3-chlorphenylessigsäure (Beispiel 1) wurde in Tetrahydrofuran mit $NaBH_4/AlCl_3$ zum entsprechenden Alkohol reduziert. Der erhaltene Alkohol (Zielprodukt) wurde im Vakuum destilliert, $Kp_{0,005}$ = 115—120° C, zeigte einen Schmelzpunkt von 40° C und wurde in einer Ausbeute von 57% erhalten.

Beispiel 7

2-Nitro-3-chlorphenylessigsäure-[2-(2-nitro-3-chlorphenyl)-ethyl]-ester

(Formel I, Y = $—CH_2C(O)—O—CH_2CH_2—$ 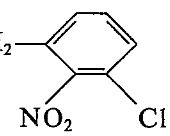 )

11,8 g 2-Nitro-3-chlorphenylessigsäure (Beispiel 1) und 10,0 g 2-(2-Nitro-3-chlorphenyl)-ethanol (Beispiel 6) wurden mit Benzol (400 ml) und einigen Tropfen konzentrierter Schwefelsäure 40 Std. am Wasserabscheider gekocht. Das Reaktionsgemisch wurde nach dem Abkühlen in der Folge mit Wasser und 2 n wäßriger Sodalösung extrahiert und die organische Phase nach Trocknung eingeengt. Der Rückstand wurde aus Isopropanol umkristallisiert und ergab 12,8 g des Zielesters, F = 72—73° C.

Beispiel 8

2-Nitro-3-chlorphenylessigsäure-2-nitro-3-chlorbenzylester

(Formel 1, Y = $—CH_2COOCH_2—$ 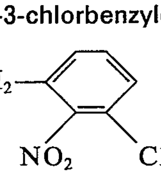 )

Nach der Arbeitsweise von Beispiel 7 wurde 2-Nitro-3-chlorphenylessigsäure (Beispiel 1) mit 2-Nitro-3-chlorbenzylalkohol, jedoch unter Verwendung einer katalytischen Menge p-Toluolsulfonsäure statt Schwefelsäure, verestert. Der Zielester, F = 103—104° C, wurde in einer Ausbeute von 83%

erhalten.

Der 2-Nitro-3-chlorbenzylalkohol (Formel 1, Y= $-CH_2OH$) wurde in ähnlicher Weise wie der Zielalkohol von Beispiel 6, jedoch aus 2-Nitro-3-chlorbenzoesäure (erhältlich gemäß Chem. Ber. 40, 3333) durch Hydrieren erhalten. Als Lösungsmittel wurde Diethylenglycoldimethyläther verwendet und das Produkt aus Tetrachlorkohlenstoff umkristallisiert, F = 62—63° C, Ausbeute 42%.

### Beispiel 9

#### 2-Nitro-3-chlorphenylessigsäure-2,4-dichlorphenylester

(Formel 1, Y = $-CH_2COO-$ 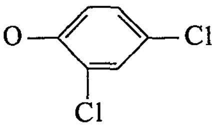 )

5 g 2-Nitro-3-chlorphenylessigsäure (Beispiel 1) wurden mit 33 ml Thionylchlorid zum 2-Nitro-3-chlorphenylessigsäurechlorid (Formel 1, Y= $-CH_2COCl$) umgesetzt, indem die Mischung von Säure und Thionylchlorid 5 Std. bei 70° C gerührt und dann bei 40° C zur Trockenheit eingeengt wurde.

Der Rückstand wurde in 20 ml trockenem Chloroform gelöst und innerhalb von 60 min bei 0° C zu einer Mischung aus 3,79 g 2,4-Dichlorphenol in 20 ml Pyridin getropft. Das Reaktionsgemisch wurde über Nacht stehengelassen, dann auf Eis gegossen, mit 10%iger HCl sauer gestellt und mit Chloroform extrahiert. Die organische Phase wurde in der Folge mit 2 n Natronlauge und mit Wasser extrahiert, getrocknet und eingeengt. Der Rückstand, der rohe Zielester, kann zur Reinigung in Chloroform auf Kieselgel durch Chromatographieren gereinigt werden, was 4 g des reinen Zielesters, F = 81—82° C, ergibt.

### Beispiel 10

#### 2-Nitro-3-chlorphenylessigsäure-2,4-dichlorbenzylester

(Formel 1, Y = $-CH_2COOCH_2-$ 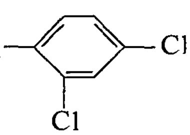 )

Dieser Zielester, F = 80—81° C, wurde in analoger Weise wie der Zielester von Beispiel 7, jedoch unter Verwendung von 2,4-Dichlorbenzylalkohol als Alkoholkomponente, hergestellt.

### Beispiel 11

#### 2-Nitro-3-chlorphenylessigsäure-(2-dimethylamino)-ethylester-hydrochlorid

(Formel 1, Y= $-CH_2COOCH_2CH_2N(CH_3)_2 \cdot HCl$)

Das Kaliumsalz der 2-Nitro-3-chlorphenylessigsäure wurde in Ethanol vorgelegt, dann das 1-Chlor-2-dimethylaminoethan in Benzol zugegeben und die Suspension 3 Std. am Rückfluß gekocht. Anschließend wurde das Reaktionsgemisch zweimal mit Wasser gewaschen, getrocknet und eingeengt. Das rotbraune Öl wurde mit methanolischer Salzsäure in das Hydrochlorid übergeführt und aus Methanol/Äther umkristallisiert, F = 150—152° C.

### Beispiel 12

#### 2-Nitro-3-chlorphenethylester der [4-(4'-Chlorbenzyl)-phenoxy]-essigsäure

(Formel 1, Y= $-CH_2CH_2-O-C(O)CH_2-O-$Phen$-CH_2-$Phen$-Cl$)

Das gemäß Beispiel 6 erhaltene 2-(2-Nitro-3-chlorphenyl)-ethanol wurde mit [4-(4'-Chlorbenzyl)-phenoxy]-essigsäure durch Kochen mit Benzol in Gegenwart einer katalytischen Menge p-Toluolsulfonsäure (Wasserabscheider) verestert. Der erhaltene Ester hat einen Schmelzpunkt von 86—87° C (Ausbeute 88%).

Die in diesem Beispiel verwendete [4-(4'-Chlorbenzyl)-phenoxy]-essigsäure der Formel

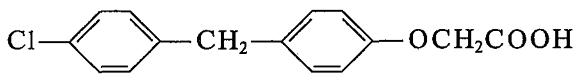

kann wie folgt hergestellt werden:
Eine Mischung aus 136,0 g (0,62 Mol) 4'-Chlor-4-hydroxy-diphenylmethan [p-(p'-Chlorbenzyl)-phenol)], 59,0 g (0,62 Mol) Chloressigsäure und 57,6 g (1,44 Mol) Natriumhydroxid in 600 ml Wasser wird während 6 Std. unter Stickstoff bei Rückflußtemperatur gerührt. Die entstandene klare Lösung, in welcher sich nach Stehenlassen bei Raumtemperatur ein kristalliner Niederschlag bildet, wird mit 5 n Salzsäure angesäuert und anschließend mit Äther erschöpfend extrahiert. Die vereinigten Ätherextrakte werden unter vermindertem Druck zur Trockene eingedampft.

Der Rückstand wird mit einer 10%igen $NaHCO_3$-Lösung behandelt, filtriert und zur Entfernung des nicht umgesetzten Phenolausgangsstoffes mehrmals mit Äther gewaschen. Dann wird das Produkt erneut mit 5 n Salzsäure angesäuert und mit Äther extrahiert. Die ätherische Lösung wird mit Wasser gewaschen, über wasserfreiem Magnesiumsulfat getrocknet und unter vermindertem Druck eingedampft. Durch Umkristallisieren aus Aceton werden 87,2 g der gewünschten Phenoxyessigsäure in Form von weißen Kristallen, F = 131 — 133° C, erhalten.

## Beispiel 13

### 2-Nitro-3-chlorphenylessigsäurehydrazid

(Formel 1, Y = $-CH_2CONHNH_2$)

Der gemäß Beispiel 4 hergestellte Methylester der 2-Nitro-3-chlorphenylessigsäure wurde mit Hydrazinhydrat umgesetzt. Die angefallene Substanz wurde aus Isopropanol umkristallisiert, F = 138 — 139° C.

## Beispiel 14

### 2-Nitro-3-chlorphenylessigsäureamid

(Formel 1, Y = $-CH_2CONH_2$)

Das gemäß Beispiel 9 (erster Absatz) hergestellte 2-Nitro-3-chlorphenylessigsäurechlorid wurde als Lösung in $CH_2Cl_2$ zu konzentriertem wäßrigem Ammoniak ($NH_4OH$) getropft. Dadurch wurde die Zielverbindung dieses Beispiels (F = 142 — 143° C) in einer Ausbeute von etwa 70% erhalten.

## Beispiel 15

### N-[2-(4,5-Dihydroimidazol)]-2-nitro-3-chlorphenyl-acetamid

(Formel 1, Y = $-CH_2CONH-$ 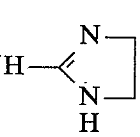 )

0,54 g metallisches Natrium wurden in 50 ml Isopropanol gelöst. Die warme Lösung wurde zu einer Suspension von 4,9 g Aminoimidazolin-hydrojodid gegeben und die entstandene klare farblose Lösung 1 Std. bei Raumtemperatur stehengelassen. Anschließend wurden 5,3 g 2-Nitro-3-chlorphenylessigsäure-methylester (Beispiel 4) portionenweise zugegeben und die violette Lösung 3 Tage bei Raumtemperatur stehengelassen. Die ausgefallene Zielsubstanz wurde abgenutscht und aus Methylenchlorid/Methanol umkristallisiert, F = 211 — 213° C.

## Beispiel 16

### N-(2-Hydroxyethyl)-2-nitro-3-chlorphenylacetamid

(Formel 1, Y= $-CH_2CONHCH_2CH_2OH$)

5 g 2-Nitro-3-chlorphenylessigsäure-methylester (Beispiel 4) wurden mit 5 ml Ethanolamin über Nacht bei Raumtemperatur gerührt, anschließend in Chloroform gelöst und mit Wasser extrahiert. Die organische Phase wurde getrocknet und teilweise eingeengt. Die dabei entstandenen Kristalle der Zielverbindung, F=97—99° C, wurden abgenutscht.

## Beispiel 17

### N-(2-Bromethyl)-2-nitro-3-chlorphenylacetamid

(Formel 1, Y= $-CH_2CONHCH_2CH_2Br$)

5 g der gemäß Beispiel 6 erhaltenen Verbindung, 1,53 g Pyridin und 15 ml Chloroform wurden vorgelegt; dann wurden bei −10° C 4,42 g $SOBr_2$ in 5 ml Chloroform innert 30 min zugetropft. Die hellrote Lösung wurde anschließend 5 Std. bei Raumtemperatur gerührt, dann auf Eis gegossen und die organische Phase mit 2 n wäßriger Sodalösung geschüttelt, getrocknet und eingeengt. Der Rückstand wurde aus Chloroform umkristallisiert. Die erhaltene Zielverbindung hatte einen Schmelzpunkt von 101—103° C.

## Beispiel 18

### N-(2-Aminoethyl)-2-nitro-3-chlorphenylacetamid

(Formel 1, Y= $-CH_2CONHCH_2CH_2NH_2$)

5 g 2-Nitro-3-chlorphenylessigsäure-methylester (Beispiel 4) wurden mit 5 ml Ethylendiamin über Nacht bei Raumtemperatur gerührt; dann wurde das überschüssige Ethylendiamin abdestilliert und der Rückstand aus Essigester umkristallisiert. Dies ergab 10 g Zielprodukt, F=98—100° C.

## Beispiel 19

### 2-Nitro-3-chlorphenylacetonitril

(Formel 1, Y= $-CH_2CN$)

130 g 2-Nitro-3-chlorphenylessigsäureamid (Produkt von Beispiel 14) wurden in 135 ml absolutem Pyridin und 1250 ml absolutem Dioxan suspendiert. Dann wurde eine Mischung aus 163 g Trifluoressigsäureanhydrid und 41 g Trifluoressigsäure innerhalb von 45 min bei 6—9° C zu der Suspension getropft. Anschließend wurde 3 Std. bei Raumtemperatur gerührt, dann mit 1250 ml Chloroform verdünnt und mit Wasser und gesättigter wäßriger NaCl-Lösung gewaschen. Die organische Phase wurde getrocknet und eingeengt. Der Rückstand wurde in Äther/Petroläther aufgenommen, filtriert und das Filtrat dann eingeengt. Der Rückstand wurde aus Isopropanol umkristallisiert und ergab 97 g des Zielproduktes, F=57—58° C.

## Beispiel 20

### 2-Nitro-3-chlorphenyl-($\alpha$-methyl)-acetonitril

(Formel 1, Y= $-CH(CH_3)CN$)

1,99 g NaOH wurden in 19 ml Wasser gelöst. Dann wurden bei einer Temperatur von 10° C 8,45 g Tetrabutylammoniumhydrogensulfat zugegeben. Der dicke Brei wurde nun zu einer Mischung aus 4,9 g der nach Beispiel 19 erhaltenen Nitrilverbindung und 7,07 g Methyljodid in 19 ml Chloroform gegeben. Anschließend wurde das Ganze bei Raumtemperatur 1 Std. gerührt, wobei sich das Reaktionsgemisch entfärbte und der pH auf 7 sank. Nun wurden die Phasen getrennt, die organische Phase getrocknet und eingeengt. Der Rückstand wurde mit Äther ausgerührt und filtriert. Das Filtrat

wurde eingeengt und aus Ethanol-Wasser (2 : 1) umkristallisiert. Dies ergab 3 g Zielprodukt, F = 51 – 52° C.

### Beispiel 21

2-(2-Nitro-3-chlornitrophenyl)-propionsäure

(Formel 1, Y = $-CH(CH_3)COOH$)

7,5 g des nach Beispiel 20 erhaltenen α-Methylacetonitrilderivates wurden mit 9,05 ml Essigsäure, 9,05 ml konzentrierter Schwefelsäure und 9,05 ml Wasser während 4 Std. auf Rückfluß erhitzt. Die Reaktionsmischung wurde dann in 90 ml Wasser gegossen, abgenutscht und aus Toluol umkristallisiert. Dies ergab 5,9 g der Zielverbindung, F = 143—144° C.

### Beispiel 22

2-Nitro-3-chlorphenyl-α-bromacetamid

(Formel 1, Y = 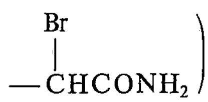 )

5 g des gemäß Beispiel 19 hergestellten 2-Nitro-3-chlorphenylacetonitrils wurden gelöst in Tetrachlorkohlenstoff mit elementarem Brom unter Zugabe einer geringen Menge einer 48%igen wäßrigen Bromwasserstofflösung bromiert. Das Zielprodukt wurde aus Methyltrichlorid umkristallisiert, F = 132—133° C, und in einer Menge von 1,4 g erhalten.

### Beispiel 23

2-Nitro-3-chlorphenyl-α-bromacetonitril

(Formel 1, Y = 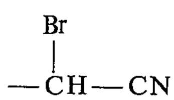 )

50 g des gemäß Beispiel 19 hergestellten 2-Nitro-3-chlorphenylacetonitrils wurden ähnlich wie in Beispiel 22, aber ohne Zugabe von wäßrigem HBr bromiert. Das Zielprodukt wurde aus i-Propanol umkristallisiert, F = 75—76° C, und in einer Menge von 24,2 g erhalten.

### Beispiel 24

(4′-Hydroxyphenyl)-2-nitro-3-chlorbenzylketon

(Formel 1, Y = 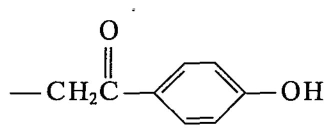 )

Das gemäß Absatz 1 von Beispiel 9 hergestellte 2-Nitro-3-chlorphenylessigsäurechlorid wurde unter den Bedingungen der Friedel-Crafts-Reaktion mit Phenol in Nitrobenzol und in Gegenwart von $AlCl_3$ bei 15—20° C umgesetzt. Das Reaktionsgemisch wurde wasserdampfdestilliert und das so erhaltene Rohprodukt zweimal aus i-Propanol umkristallisiert. Ausgehend von 86 g des Säurechlorides wurden 7,2 g Zielprodukt, F = 182—184° C, erhalten.

### Anwendungsbeispiel

Herbizidzubereitungen wurden aus jeweils

10% Verbindung der Formel (1)
20% Tensid (z. B. »Atlox® 4851B« der Firma

Atlas Chemie, BRD; »Atlox« ist ein Warenzeichen)
70% organisches Lösungsmittel, z. B. Cyclohexanon

hergestellt und in verschiedenen Dosierungen (Angaben bezogen auf Wirkstoff, a. i. = active ingredient in kg/ha) an monokotylen (Hühnerhirse) und dikotylen Testpflanzen (Tomate und Kresse) auf Herbizidwirkung durch Vorlaufbehandlungen (Pre-emergence I: Vorlaufbehandlung auf dem Pflanzensamen und anschließendes Abdecken mit Erde; Pre-emergence II: Vorlaufbehandlung auf abgedecktem Pflanzensamen) und Nachlaufbehandlungen (Post-emergence: Zweiblattstadium der Testpflanzen) untersucht.

Die Bewertungsergebnisse sind als Zahlen von 1—5 wie folgt angegeben:

1 = 0% Schädigung
2 = 25% Schädigung
3 = 50% Schädigung
4 = 75% Schädigung
5 = 100% Schädigung

Wirkungen entsprechend Bewertungszahlen von 4—5 gelten als sehr gut, solche von 3,5 bis 3,9 als gut und solche von 3 bis 3,4 als befriedigend. Werte von unter 3 entsprechen der Bewertung wirkungslos. Die Ergebnisse sind in der folgenden Tabelle für die Dosierung entsprechend 1 kg a. i./ha angegeben.

Tabelle I

| Verbindung der Formel (1) gemäß Beispiel | Pre-em. I Monokotyle | Dikotyle | Pre-em. II Monokotyle | Dikotyle | Post-em. Monokotyle | Dikotyle |
|---|---|---|---|---|---|---|
| 1 | 3.3 | 4.4 | 2.7 | 4.5 | 1.3 | 3.9 |
| 2 | 3.0 | 5.0 | 1.0 | 4.9 | 1.7 | 4.3 |
| 3 | 4.0 | 5.0 | 4.0 | 5.0 | 1.3 | 3.8 |
| 4 | 3.3 | 4.8 | 3.8 | 5.0 | 1.3 | 3.9 |
| 5 | 3.0 | 4.5 | 3.0 | 4.4 | 1.0 | 4.0 |
| 6 | 2.3 | 4.7 | 2.3 | 4.8 | 1.5 | 2.6 |
| 7 | 3.8 | 5.0 | 3.8 | 4.8 | 1.0 | 3.9 |
| 8 | 2.8 | 4.8 | 2.8 | 4.8 | 1.3 | 3.9 |
| 9 | 3.0 | 4.5 | 3.8 | 5.0 | 1.0 | 3.7 |
| 10 | 2.8 | 4.8 | 3.3 | 4.8 | 1.0 | 4.0 |
| 11 | 2.8 | 4.4 | 2.8 | 4.6 | 1.5 | 3.9 |
| 12 | 2.0 | 4.3 | 2.0 | 4.7 | 1.0 | 1.3 |
| 13 | 3.3 | 4.8 | 3.0 | 4.9 | 1.0 | 3.2 |
| 14 | 3.8 | 4.9 | 3.0 | 4.9 | 1.0 | 2.7 |
| 15 | 2.0 | 3.6 | 2.8 | 4.7 | 1.0 | 3.5 |
| 16 | 2.8 | 4.5 | 2.8 | 4.4 | 1.0 | 2.2 |
| 17 | 1.3 | 3.9 | 1.3 | 3.8 | 1.0 | 2.9 |
| 18 | 1.7 | 3.8 | 1.7 | 2.2 | 1.0 | 2.6 |
| 19 | 3.3 | 5.0 | 3.3 | 5.0 | 1.5 | 2.8 |
| 20 | 2.0 | 4.5 | 2.0 | 3.9 | 1.0 | 1.9 |
| 21 | 2.0 | 4.5 | 2.0 | 4.5 | 1.0 | 2.5 |
| 24 | 2.0 | 4.4 | 2.0 | 2.9 | 1.0 | 2.5 |

Allgemein können Herbizide mit den aktiven Komponenten (1) in üblichen Dosierungen von 0,1 bis 10 kg Wirkstoff/ha zur selektiven oder generellen Bekämpfung unerwünschter Vegetation in Landwirtschaft oder Gartenbau in den üblichen Zubereitungsformen, wie Spritzpulver, Granulate, Mikrogranulate, Lösungen, Dispersionen bzw. Emulsionen, mit festen oder flüssigen Trägern, Hilfsstoffen, wie Tensiden oder Antischaummitteln, gegebenenfalls in Mischung untereinander oder kombiniert mit bekannten Herbiziden bzw. anderen agrochemischen Mitteln, wie Pestiziden und Dünger, verwendet werden. Der Gehalt erfindungsgemäßer Herbizide an Verbindung(en) der Formel (1) kann allgemein zwischen 1 und 99% liegen und beträgt meist 10—50%. Selbstverständlich können erfindungsgemäße Herbizide auch als Konzentrate zur nachträglichen Verdünnung mit flüssigen oder festen Streckmitteln am Ort des Verbrauches hergestellt werden.

**Patentansprüche**

1. Verwendung von Verbindungen der Formel (1)

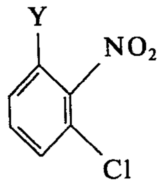

(1)

als selektive Herbizide, wobei in Formel (1) Y eine Gruppe der Formeln (1a), (1b) oder (1c)

$—CH_2CH_2—OR^1$ 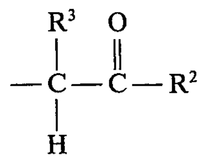 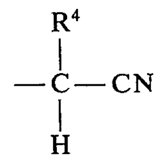

(1a) (1b) (1c)

darstellt, in welchen

$R^1$ das Wasserstoffatom oder der Säurerest einer $C_{1-6}$-Alkansäure ist, die gegebenenfalls einen $C_{6-12}$-Aryl-, $C_{6-12}$-Aryloxy-, $C_{6-12}$-Ar-$C_{1-18}$-alk-$C_{6-12}$-aryl- oder $C_{6-12}$-Ar-$C_{1-18}$-alk-$C_{6-12}$-$C_{6-12}$-aryloxyrest trägt, dessen Arylteil durch Halogen und/oder Nitro substituiert sein kann;

$R^2$ das Wasserstoffatom; die Gruppe —OZ, in welcher Z das Wasserstoffatom, den einwertigen Rest eines Kations oder einen gegebenenfalls durch Halogen und/oder Nitro substituierten $C_{1-18}$-Alkyl-, $C_{6-12}$-Aryl-, $C_{7-18}$-Alkaryl- oder $C_{7-18}$-Aralkylrest bedeutet; eine Gruppe der Formel

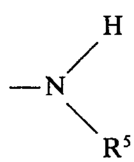

in welcher $R^5$ das Wasserstoffatom, die Aminogruppe, einen gegebenenfalls durch Halogen oder Nitro substituierten $C_{1-18}$-Alkylrest oder einen alicyclischen oder heteroaromatischen Rest mit 5—6 Ringatomen darstellt; oder ein gegebenenfalls durch Halogen und/oder Nitro substituierter $C_{1-18}$-Alkyl-, $C_{6-12}$-Aryl-, $C_{7-18}$-Alkaryl- oder $C_{7-18}$-Aralkylrest ist; und

$R^3$ und $R^4$ Wasserstoffatome, Niederalkylgruppen ($C_{1-4}$) oder Halogenatome sind.

2. Neue Verbindungen der Formel (1)

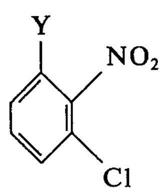

(1)

in welcher Y eine Gruppe der Formeln (1a), (1b) oder (1c)

$—CH_2CH_2—OR^1$ 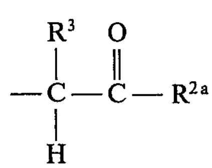 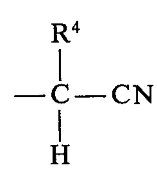

(1a) (1b) (1c)

darstellt, in welchen

$R^1$ das Wasserstoffatom oder der Säurerest einer $C_{1-6}$-Alkansäure ist, die gegebenenfalls einen

$C_{6-12}$-Aryl-, $C_{6-12}$-Aryloxy-, $C_{6-12}$-Ar-$C_{1-18}$-alk-$C_{6-12}$-aryl-, oder $C_{6-12}$-Ar-$C_{1-18}$-alk-$C_{6-12}$-aryloxyrest trägt, dessen Arylteil gegebenenfalls durch Halogen und/oder Nitro substituiert ist;

$R^{2a}$ die Aminogruppe oder eine N-Monoalkylaminogruppe ist, deren Alkylteil 1—6 C-Atome enthält und gegebenenfalls durch Amino, Halogen oder Hydroxyl substituiert ist; und

$R^3$ und $R^4$ Wasserstoffatome, Niederalkylgruppen ($C_{1-4}$) oder Halogen sind.

## Claims

1. Use of compounds of the formula (1)

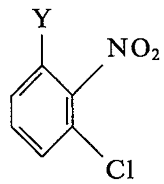

(1)

as selective herbicides, in which formula (1) Y is a group of formula (1a), (1b) or (1c)

$—CH_2CH_2—OR^1$ 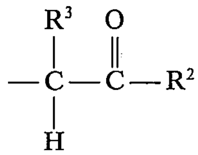 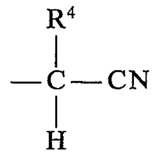

(1a) (1b) (1c)

in which

$R^1$ is the hydrogen atom or the acid residue of a $C_{1-6}$-alcanoic acid optionally carrying a $C_{6-12}$-aryl, $C_{6-12}$-aryloxy, $C_{6-12}$-ar-$C_{1-18}$-alk-$C_{6-12}$-aryl or $C_{6-12}$-ar-$C_{1-18}$-alk-$C_{6-12}$-aryloxy residue, the aryl moiety of which may be substituted by halogen and/or nitro;

$R^2$ is the hydrogen atom, the group —OZ, in which Z is the hydrogen atom, the monovalent residue of a cation or a $C_{1-18}$-alkyl, $C_{6-12}$-aryl, $C_{7-18}$-alkaryl, or $C_{7-18}$-aralkyl residue optionally substituted by halogen and/or nitro; a group of the formula

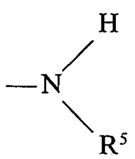

in which $R^5$ is the hydrogen atom, the amino group, a $C_{1-18}$-alkyl residue optionally substituted by halogen or nitro, or an alicyclic or heteroaromatic residue having 5—6 ring atoms; or a $C_{1-18}$-alkyl, $C_{6-12}$-aryl, $C_{7-18}$alkaryl or $C_{7-18}$-aralkyl residue optionally substituted by halogen and/or nitro; and

$R^3$ and $R^4$ are hydrogen atoms, lower alkyl groups ($C_{1-4}$) or halogen atoms.

2. Novel compounds of the formula (1)

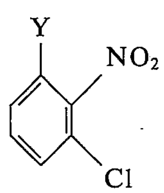

(1)

in which Y is a group of the formula (1a), (1b) or (1c)

$-CH_2CH_2-OR^1$ 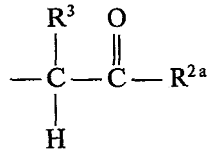 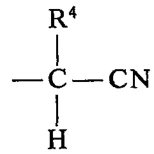

(1a) (1b) (1c)

in which

$R^1$ is the hydrogen atom or the acid residue of a $C_{1-6}$-alcanoic acid optionally carrying a $C_{6-12}$-aryl, $C_{6-12}$-aryloxy, $C_{6-12}$-ar-$C_{1-18}$-alk-$C_{6-12}$-aryl or $C_{6-12}$ar-$C_{1-18}$-alk-$C_{6-12}$-aryloxy residue, the aryl moiety of which is optionally substituted by halogen and/or nitro;

$R^{2a}$ is the amino group or an N-monoalkyl amino group, the alkyl moiety of which contains from 1 to 6 C-atoms and is optionally substituted by amino, halogen or hydroxyl; and

$R^3$ and $R^4$ are hydrogen atoms, lower alkyl groups ($C_{1-4}$) or halogen.

**Revendications**

1. Utilisation de dérivés de formule (1)

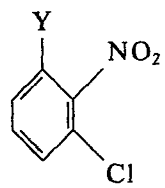

(1)

en tant qu'herbicides sélectifs, caractérisée en ce que dans la formule (1) Y représente un groupe de formules (1a), (1b) ou (1c)

$-CH_2CH_2-OR^1$ 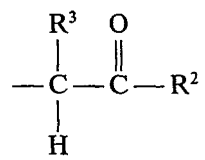 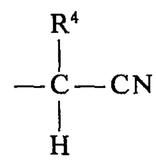

(1a) (1b) (1c)

dans lequel,

$R^1$ représente un atome d'hydrogène ou un reste acide d'un acide alcanoique en $C_1-C_6$ qui porte éventuellement un groupe aryle en $C_6-C_{12}$, aryloxy en $C_6-C_{12}$, aryl ($C_6-C_{12}$)-alkyl ($C_1-C_{18}$)-aryle ($C_6-C_{12}$) ou aryl ($C_6-C_{12}$)-alkyl ($C_1-C_{18}$)-aryloxy-($C_6-C_{12}$) dont la fraction aryle peut être substituée par un ou plusieurs atomes d'halogène et/ou groupes nitro;

$R^2$ représente un atome d'hydrogène; un groupe $-OZ$, Z représentant un atome d'hydrogène, le reste monovalent d'un cation ou un groupe alkyle en $C_1-C_{18}$, aryle en $C_6-C_{12}$, alkylaryle $C_7-C_{18}$ ou arylalkyle en $C_7-C_{18}$, éventuellement substitué par un ou plusieurs atomes d'hydrogène et/ou groupes nitro; un groupe de formule

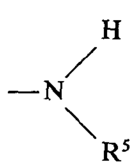

dans lequel $R^5$ représente un atome d'hydrogène, un groupe amino, un groupe alkyle en $C_1-C_{18}$, éventuellement substitué par un ou plusieurs atomes d'halogène ou groupes nitro ou un reste alicyclique ou hétéroaromatique comportant 5 à 6 atomes de cycle; ou un groupe alkyle en $C_1-C_{18}$, aryle en $C_6-C_{12}$, alkylaryle en $C_7-C_{18}$ ou arylalkyle en $C_7-C_{18}$, éventuellement substitué par un ou plusieurs atomes d'halogene et/ou groupes nitro; et

$R^3$ et $R^4$ représentent des atomes d'hydrogène, des groupes alkyles inférieurs en $C_1-C_4$ ou des atomes d'halogène.

2. Nouveaux dérivés de formule (1)

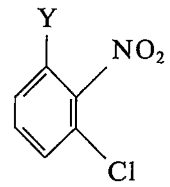

(1)

dans laquelle Y représente un groupe de formules (1a), (1b) ou (1c)

$—CH_2CH_2—OR^1$ 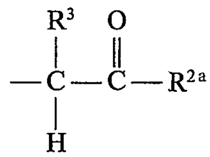 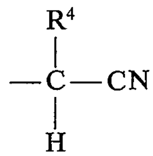

(1a) (1b) (1c)

dans lequel,

$R^1$ représente un atome d'hydrogène ou un reste acide dans un acide alkanoique en $C_1—C_6$ qui porte éventuellement un groupe aryle en $C_6—C_{12}$-aryloxy en $C_6—C_{12}$, aryl ($C_6—C_{12}$)-alkyl $C_1—C_{18}$)-aryle ($C_6—C_{12}$) ou aryl ($C_6—C_{12}$)-alkyl ($C_1—C_{18}$)-aryloxy ($C_6—C_{12}$) dont la fraction aryl peut être substituée par un ou plusieurs atomes d'halogène et/ou un groupe nitro;

$R^{2a}$ représente un groupe amino ou N-aminoalkylamino dont la fraction alkyle contient 1 à 6 atomes de carbone et est éventuellement substituée par un ou plusieurs groupes amino ou hydroxy ou atomes d'halogène; et

$R^3$ et $R^4$ représentent des atomes d'hydrogène, des groupes alkyles inférieurs en $C_1—C_4$ ou des atomes d'halogène.